# EUROPEAN PATENT SPECIFICATION

(11) **EP 1 414 475 B1**
(45) Date of publication and mention of the grant of the patent: **19.01.2005**
(21) Application number: 02760548.4
(22) Date of filing: 09.08.2002
(51) Int. Cl.: A61K 35/78, A61P 11/00

(54) **AN HERBAL COMPOSITION FOR THE TREATMENT AND REMEDY OF BRONCHIAL RESPIRATORY DIFFICULTIES**
PFLANZENPRÄPARAT ZUR BEHANDLUNG UND HEILUNG VON BRONCHIALEN RESPIRATORISCHEN SCHWIERIGKEITEN
COMPOSITION VEGETALE POUR LE TRAITEMENT DES DIFFICULTES RESPIRATOIRES BRONCHIQUES

(30) Priority: 10.08.2001 US 925415; 01.11.2001 US 985160
(43) Date of publication of application: 06.05.2004
(73) Proprietor: Council of Scientific and Industrial Research, New Delhi 110 001 (IN)
(72) Inventor: BANDYOPADHYAY, Santu, Indian Inst. of Che. Bio., 700 032 Kolkata (IN); ROY, Keshab C., Indian Inst. of Che. Bio., 700 032 Kolkata (IN); ROY, Mitali, Indian Inst. of Che. Bio., 700 032 Kolkata (IN); PAL, Bikash C., Indian Inst. of Che. Bio., 700 032 Kolkata (IN); BHADRA, Ranjan, Indian Inst. of Che. Bio., 700 032 Kolkata (IN); DAS, Krishna, Indian Inst. of Che. Bio., 700 032 Kolkata (IN); BHATTACHARAYA, Samir, Indian Inst. of Che. Bio., 700 032 Kolkata (IN)
(74) Representative: RACKETTE Partnerschaft Patentanwälte
(86) International application number: PCT/IN2002/000166
(87) International publication number: WO 2003/013564

(56) References cited:
- US-A1- 2002 086 068
- US-B1- 6 413 553
- SINGH Y N: "TRADITIONAL MEDICINE IN FIJI: SOME HERBAL FOLK CURES USED BY FIJI INDIANS" JOURNAL OF ETHNOPHARMACOLOGY, ELSEVIER SCIENTIFIC PUBLISHERS LTD, IE, vol. 15, no. 1, 1986, pages 57-88, XP001000897 ISSN: 0378-8741
- SOMANADHAN B. ET AL.: "An ethnopharmacological survey for potential angiotensin converting enzyme inhibitors from Indian medicinal plants." JOURNAL OF ETHNOPHARMACOLOGY, vol. 65, no. 2, 1999, pages 103-112, XP001121516 Elsevier Scientific publishers LTD, IE
- PATEL R.S. , RAJORHIA G.S.: "Antioxidative Role of Curry (Murraya koenigi) and Betel (Piper betel) leaves in ghee" JOURNAL OF FOOD SCIENCE AND TECHNOLOGY, vol. 16, July 1979 (1979-07), pages 158-160, XP009002758

## Description

### Technical Field

This invention relates to an herbal composition for the treatment and remedy of bronchial respiratory difficulties. This invention relates to an herbal formulation useful for blocking of 5-lipoxygenase activity leading to the inhibition of leukotrine synthesis, suppression of IL4 production and enhancement of IFN gamma release. More particularly this invention describes the process of separation, physicochemical characterization and biological response evaluation of extracts or bioactive fractions obtained from extracts of any plant parts including leaves, barks, roots and seeds of plants *M*. *koenigii* and *P. betle* in order to establish their role on the treatment and remedy of bronchial respiratory troubles.

### Background Art

Respiratory problem consists of mild to extremely severe trouble of breathing along with the other discomforts such as wheezing, coughing, chest tightness and the like. In spite of precautionary measure, public awareness campaign and monitoring system, population having respiratory trouble is on the rise all over the world. This is true in western advanced countries, especially among the children. Using *M*. *koenigii* leaf preparation, the relief and possible cure of asthma has already been demonstrated by the applicants in their PCT Patent application No: PCT/IN/00102 filed on dated Oct. 16^{th}, 2000. Two-prong strategy is adapted in the present invention.

The respiratory disease is the result of pathophysiological symptoms arising out of aberration of immune system. The immediate symptom includes bronchial constriction, inflammation of respiratory tract and closing of air way by mucus secretion. The symptomatic drugs provide relief by temporary relaxation of the distressed symptoms.

This is to state that the *Murrya koenigii* extract combined with extract obtained from *Piper betle* has shown surprising results. According to the earlier filed unpublished PCT patent application, the betle leaf extract showed increase in IFN-γ from Th1 type cells and decrease of IL-4 from Th2 type cells. This clearly is an effective measure in treating asthma as shifting of Th2 type response towards Th1 type will obviously reduce the release of IgE from mast cells which is a major manipulator of asthmatic condition. Hence the applicants earlier finding followed by the filing of PCT patent application PCT/INOO/00127 is in direct consonance with the present filing on combined extracts from any plant parts of *M. koenigii* and *P. betle* as a new medicine for treating atopic Asthma.

The root cause for respiratory problems is not well addressed by the developers of symptomatic drugs. With the advent of current knowledge, it is now well accepted that lcukotrienes are found to be the main player in developing symptoms of respiratory problems.

The major symptoms of respiratory problem can be divided into early and late responses. The early response occurs within minutes of allergen exposure and involves primarily mediators such as histamine, leukotrienes and prostaglandin D2. The effects of these mediators result in bronchocontriction and accumulation of mucus. The late response occurs hours later and involves additional mediators including IL-4, IL-5, IL-6, and TNF-alpha, eosinophils chemotactic factor (ECF) and platelet aggregation factor (PAF). The overall effect of these mediators is to recruit inflammatory cells including eosinophils and neutrophils. These cells capable of causing significant tissue injury by releasing toxic enzymes. These events lead to occlusion of bronchial lumen with mucus protein, and cellular debris, thus thickening of basement membrane, fluid build up and hypertrophy of the bronchial smooth muscle. A mucus plug often forms and adheres to the bronchial wall. The mucus plug contains clusters of detached epithelial cells fragments, eosinophils, some neutrophils and spirals of bronchial tissue known as Curschmann's spiral (Immunology, J. Kuby; W.H. Freeman & Co., New York; 3rd edition 1997). In view of the current mechanisms regarding manifestation of bronchial asthma/ bronchial respiratory problems, modern strategy for drug development stressed the following approaches:

These include i) inhibition of leukotriene synthesis via blocking the 5-fipoxygenase-enzyme activity (Leqqis RA et al New England J. Med. 323:, 645,1990). The formation of leukotrienes originates from the oxidation of arachidonic acid, hence inhibition of this reaction leads to the inhibition of leukotriene synthesis. Besides, leukotriene receptors antagonists have also been introduced as anti leukotriene therapy for asthma/respiratory problems (Tien F.C., Medical J. Aust. 171: 378,1999). Currently licensed drug zelutin, based on inhibition of arachidonic acid oxidation has already been introduced exclusively in the US market. However, its use is limited by hepatotoxicity. Children below 14 years are not recommended for this drug. Moreover, patients taking other drugs need to be surveyed when taking zelutin as anti-asthma drug. More over patients taking other drugs, need to be surveyed when taking zelutin, as anti-asthma drug. ii) the neutralization of IgE either by anti-IgE antibody (humanized) or by blocking the high affinity IgE receptor, FcεR-I (Heusser C, Jardiu P. Current Oppinio Immunol., 9: 805, 1999). Since suppression of Th2 cytokines leads to decrease in IgE production, additional approach is based on the inhibition of these Th2 cytokine synthesis and enhancement of Th1 cytokine formation (Chung K.F.; Barens P.J. Thorax 54: 825, 1999).

In contrast, *M. koenigii* based anti asthmatic preparation has been tried on children as young as 7 years old and octagenarian as old as 80 years and above without any adverse reaction.

The extracts as well the fractions of *M*. *koenigii* extracts obtained from all plant parts including leaves, barks, roots and seeds are therefore examined to identify the bioactive fractions. These are based on their potential to inhibit the production of leukotrienes. Additionally, the fractions are also examined for shifting of Th2 response towards to Th1 type. Th1 and Th2 response are measured by γ-interferon (Th1) and IL-4 (Th2) production respectively. Some *M*. *koenigii* fractions showed inhibition of 5-lipoxygenase mediated arachidonic acid oxidation signifying blockage of leukotriene synthesis and remarkable increase in γ-interferon production which in turn would suppress IL-4 production. On the other hand, most of the *P. belle* leaf extract-fractions resulted in facilitating of the shift from Th2 type response to Th1 type. Thus, a blend of selected extract fractions from these two plants *M. koenigii and P. betle* predominantly inhibited leukotriene synthesis and shifted Th2 response towards Th1 type and therefore is proposed as a unique synergistic composition for treatment, relief and remedy of bronchial respiratory problems Thus the two pronged strategy is the major objective of this new composition and can be considered as the best modality of treatment for patients bronchial respiratory problems.

### Objects of the Invention

The main object of the invention is to provide the bioactive fractions, which are obtained from extracts of the leaves of *Murraya koenigii* and *Piper betle.*

Another object of the invention is to provide a new phannaceutical composition comprising combination of extracts derived from leaves of Murraya *koenigii* and *Piper betle.*

Another object of the invention is to provide a new composition comprising combination of bioactive fractions derived from *Murraya koenigii* and *Piper belle* plant parts for treating respiratory problems.

Yet another objective of the present invention is to provide a process for the preparation of extract from the leaves or any other plant parts of *M. koenigii* and *P. betle* useful for relief, treatments and remedy of respiratory problem.

Another object of the present invention is to provide a process for the isolation of bioactive fractions from the plant parts of *M*. *koenigii* and *P. betle* useful for relief, treatments and remedy of respiratory problems.

Yet another objective of the present invention is to provide a simplified method of isolation of bioactive fractions from all plant parts of *M*. *koenigii* and *P. betle* possessing biological activities relevant to treatment, relief and remedy of respiratory problems.

Yet another objective of the present invention is to provide a herbal preparation comprising of extracts derived from *M*. *koenigii* and *P*. *betle* leaves or extracts obtained from other plant parts of *M*. *koenigii* and *P. betle*, wherein the said extracts being highly compatible for human consumption and capable for being used for the treatments, relief and remedy of respiratory problem.

Yet, another objective of the present invention is to provide a simplified fast and inexpensive process for the preparation of a composition possessing biological activities relevant to treatments, relief and remedy of respiratory problems.

Yet another objective of the present invention is to provide an herbal composition preparation, comprising of active factors and bioactive fractions derived from all plant parts of M. *koenigii* and *P. betle,* wherein the said factors and bioactive fractions being highly compatible for human consumption and the treatments, relief and remedy of respiratory problems.

Yet another objective of the present invention is to examine each of the bioactive fractions individually or in combination from all plant parts *M. koenigii* and *P. betle* leaves having inhibitory activity of 5-lipoxygenase mediated Arachidonic acid oxidation and promoting the shift of Th2 type response towards Th1 type.

Yet another objective of the present invention is to examine each of the extracts obtained from *M. koenigii* and *P. betle* leaves or other plants parts individually and in combination on the inhibitory activity of 5-lipoxygenase-mediated Arachidonic acid oxidation and inducing the shift of Th2 type response towards Th1 type.

Yet another objective of the present invention is to assay 5-lipoxygenase mediated Arachidonic acid oxidation in an *ex vivo* whole human blood system in the presence of *M*. *koenigii* and *P. betle* fractions individually and in combination.

Yet another objective of the present invention is to assay the 5-lipoxygenase mediated Arachidonic acid oxidation in *ex vivo* systems with enriched human polymorphonuclear neutrophils (PMN) in the presence of *M*. *koenigii* and *P. betle* fractions individually and in combination.

Yet another objective of the present invention is to assay 5-lipoxygenase mediated Arachidonic acid oxidation in an *ex vivo* human whole blood system in the presence of *M*. *koenigii* and *P*. *betle* leaves extracts or their other plant part extracts individually and in combination.

Yet, another objective of the present invention is to detect the production of intracellular cytokines by flowcytometry.

Yet another objective of the present invention is to provide intracellular IFN-gama and IL-4 profile as known markers for Th1 and Th2 type response respectively in *ex vivo* human whole blood with the fractions from all plant parts of *M. koenigii* and *P. betle* individually and in combination

### Summary of the invention

In accordance, the present invention provides an herbal formulation useful for blocking of 5 lipoxygenase activity leading to the inhibition of leukotrine synthesis, suppression of IL4 production and enhancement of IFN gamma release

In addition, the present invention provides an herbal fonnulation for the treatment and remedy of bronchial respiratory difficulties. The invention also provides a process for the preparation of extracts from the leaves or any other plant parts or seeds of plants *M*. *koenigii* and *P. betle* and their selective blending to evaluate biological response in order to establish their role for the treatment and remedy of bronchial respiratory troubles.

In addition, the present invention provides an herbal composition comprising bioactive fractions obtained from the leaves or any other plant parts or seeds of plants *M*. *koenigii* and *P. betle* and their selective blending for the treatment and remedy of bronchial respiratory difficulties. Another aspect of the invention, relates to a process of separation, physicochemical characterization and biological response evaluation of bioactive fractions obtained from the extracts of any plant parts including leaves, barks, roots and seeds of plants *M. koenigii* and *P. betle* in order to establish their role on the treatment and remedy of bronchial respiratory troubles.

### Brief description of the accompanying drawings

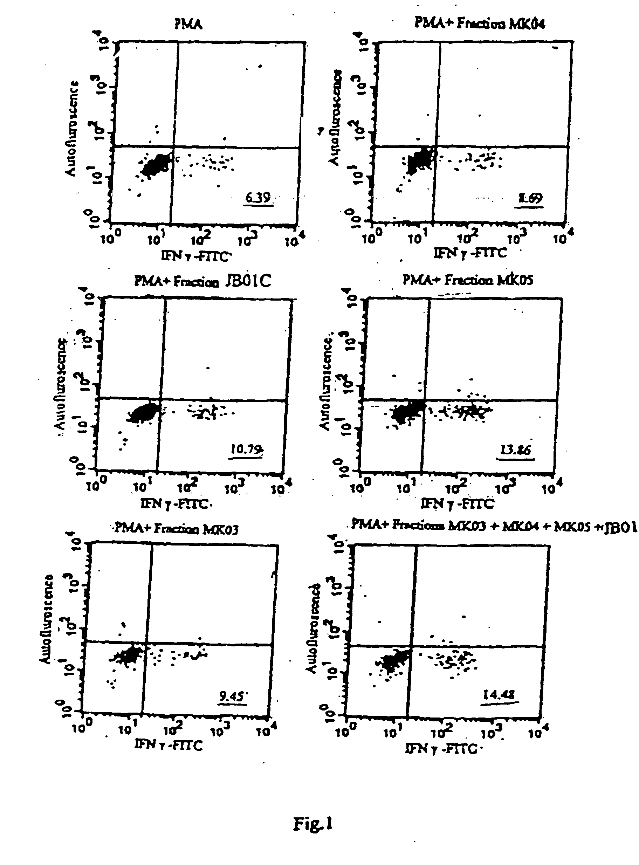

**Fig.1** describes the flow-cytometric determination of intracellular IFN-gamma in *ex vivo* human mononuclear cells after co-culture with phorbol mysistate acetate (PMA) and calcium ionophore (ionomycin) in the presence or absence of bioactive fractions obtained from *M*. *Koenigii* (MK03, MK04, MK05) and *P.betle* (JB01C). Our data indicate that bioactive fractions MK03, MK04, MK05 and JB01C enhanced IFN-gamma production.

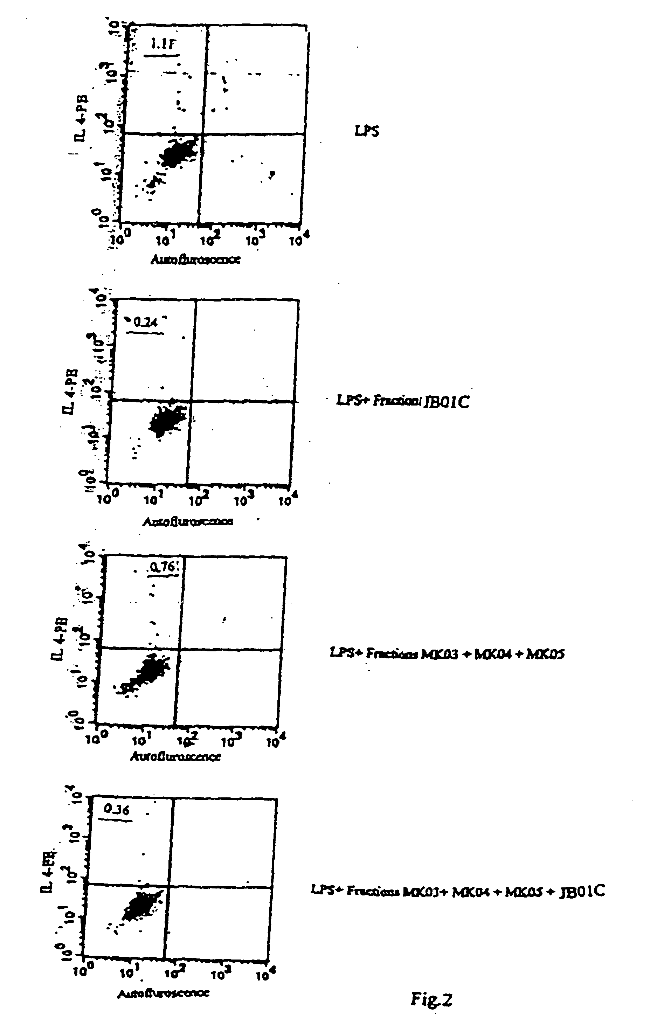

**Fig.2** describes flow-cytometric determination of intracellular IL-4 in *ex vivo* human neutrophils after coculture with LPS in the presence or absence of fractions MK03, MK04, MK05 and JB01C. Our data indicate that fraction JB01C of *P.betle* reduces IL-4 producing cells markedly. On the other hand, fractions MK03, MK04, MK05 of *Murraya koenigii* had only marginal effects on inhibition of IL-4 production. Of interest, combination of bioactive fractions MK03, MK04, MK05 of *Murraya koenigii* and bioactive fraction JB01C of *P*.*betle* drastically reduces IL-4 production.

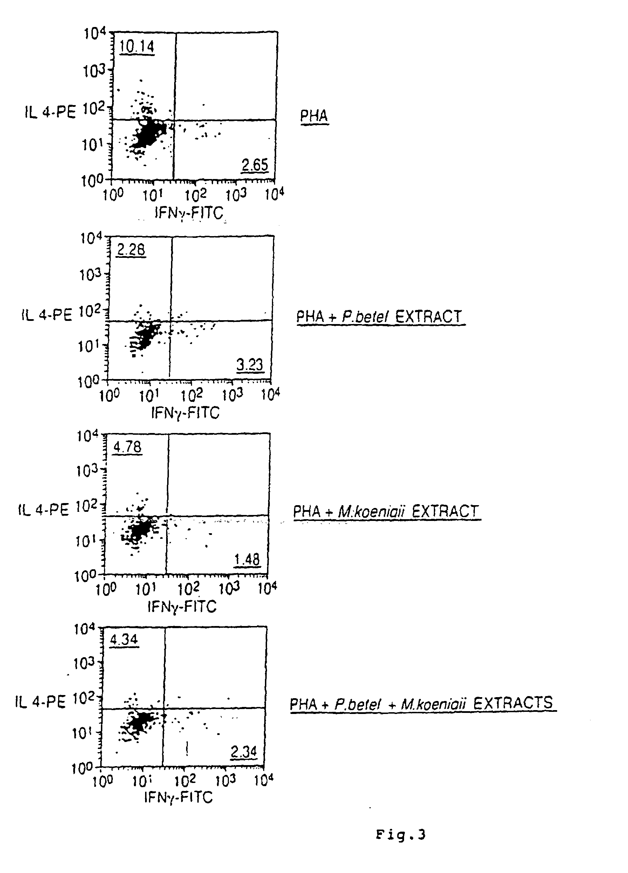

**Fig 3** describes the flow cytometric determination of intracellular IFN-gamma and IL-4 in ex vivo human mononuclear cells after co-culture with phytohemmaglutinin in the presence or absence of extracts obtained from *M*. *Koenigii* and *P.betle.* Our data indicate that the extract from P. betle increased IFN-gamma positive cells and reduced IL-4 positive cells markedly. M. *Koenigii* extract had reduced IL-4 positive as well as IFN-gamma positive cells. Combination of these two extracts increased IFN-gamma positive cells to the control level and still maintained reduced level of IL-4 positiity.

### Detailed description of the invention

Accordingly, the present invention provides a pharmaceutical composition useful as leukotrine inhibitor, IL4 synthesis inhibitor and as Th1 type immunomudulator, the said composition comprising effective amounts of lyophilized aqueous or alcoholic extract obtained from the leaves of *Piper betle (PB)* and *Murrya koenigii (M*.*K)* or bioactive fractions derived from these extracts, with optionally one or more pharmaceutically acceptable ingredients.

In an embodiment, the bioactive fractions obtained from the plant *Murrya koenigii (M*.*K)* are designated as MK03, MK04 and MK05.

Another embodiment of the invention, the bioactive fractions obtained from the plant *Piper betle (PB)* are designated as JB01A, JBO1B and JB01C.

Still another embodiment relates to the pharmaceutically acceptable ingredients used, which is selected from nutrients such as proteins, carbohydrates, sugar, talc, magnesium stearate, cellulose, calcium carbonate, starch-gelatin paste and/or carriers, excipient, diluent or solvent.

Still another embodiment relates to administering the said composition through inhalation, oral, intravenous, intramuscular, subcutaneous routes or any other suitable routes.

Yet another embodiment, the oral route is in the form of capsule, syrup, concentrate, powder or granules and the amount administered by oral route is more than intravenous route.

Yet another embodiment, the proportion of *M*.*K* extract is equal to or greater than the amount of *piper betle* extract.

Yet another embodiment, the ratio of *piper betle* (**PB**) extract to *M*.*K* extract is in the range of 1: 1 to 1:5.

Yet another embodiment, the composition is administered at a dosage level between I to 20 mg/kg of body weight at least once in a day for a period of at least 4 weeks.

Yet another embodiment, the composition consists of bioactive fractions MK03, MK04 and/or MK05.

In yet another embodiment, the said composition consists of bioactive / fractions JB01C, M K03, MK04 and/or MK05.

In yet another embodiment, the amount of bioactive fractions administered by intravenous route is less than the oral route.

In yet another embodiment, the proportion of bioactive fractions from *M*.*K* is equal to or greater than the bioactive fraction of *piper betle*.

In still another embodiment, the ratio of bioactive fractions obtained from betle extract to the bioactive fractions obtained from *M*.*K* extract is in the range of 1:1 to 1:5.

In another embodiment, the composition is administered at a dosage level between 0.5 to 10.0 mg/kg of body weight at least once in a day for a period at least 4 weeks depending upon the respiratory conditions.

In another embodiment of the invention provides a composition, which is administered for a period for at least 4 weeks and up to 3 months.

In yet another embodiment relates to a composition, which is a combination of bioactive fractions MK03, MK04 and MK05 obtained from the leaves of *Murrya Koenigi* for blocking of 5 lipooxygenase activity leading to the inhibition of leukotriene synthesis, suppression of interleukin - 4 production and as Th1 type immunomudulator.

In one more embodiment provides a composition, which is a combination of bioactive fractions MK03, MK04 and MK05 obtained from the leaves of *Murrya Koenigi and* JB01C obtained from the leaves of piper betle for blocking of 5 lipooxygenase activity leading to the inhibition of leukotriene synthesis, suppression of interleukin -4 production and for enhancement of gamma interferon release

Still another embodiment, the said composition is used for the treatment of bronchial respiratory conditions.

Still another embodiment, the said composition is used for treating mammals and animals.

Still another embodiment, the said composition is used for shifting Th2 response to Th response.

Yet another embodiment, the said composition is used for inhibiting 5-lipooxygenase mediated Arachidonic acid oxidation in neutrophils enriched component of whole blood.

Yet another embodiment, the said composition is used for enhancing IFN-gamma and reducing IL-4 response in ex-vivo human whole blood.

Yet another embodiment, the said composition is used for enhancing IFN-gamma response in ex vivo human whole blood mononuclear (PMN)

Yet another embodiment, the said composition is used for reducing IL-4 response in human peripheral whole blood mononuclear cells.

One more embodiment of the present invention provides an herbal composition, useful for blocking of 5 lipoxygenase activity leading to the inhibition of leukotrine synthesis, suppression of IL4 production and enhancement of IFN gamma release said composition comprising effective amount of extracts obtained from the leafs of *Piper betel and Murrya koenigii* or lyophilized extracts of *Piper betel (PB)* and *Murrya koenigii (M*.*K)*.

One more embodiment of the invention relates to a composition, wherein the bioactive fraction designated as MK03 obtained from the leaves of Murrya Koenigi for blocking of 5 lipooxygenase activity leading to the inhibition of leukotriene synthesis, suppression of interleukin -4 production having the following physicochemical characteristics:
a) the dried solid, melting point 98-100°C soluble in DMSO;
b) Thin layer chromatography shows single spot having Rf 0.48 in one of the solvent systems-chloroform and methanol (19:1);
c) The HPLC analysis shows single peak with retention time 8.5 min. at a flow rate of 0.5 ml/min using intersel ODS-3 (4.6 x 250 mm) analytical column, solvent system methanol and detection is carried out at 210 nm. HPLC: Column ODS-3 (4.6 x 250 mm) Flow rate - 0.5 ml/min; Peak at 210 nm; Retention time-3.5 min.; Solvent system - methanol.
d) ¹³CNMR, ppm (125 MH₂, CDCl₃): 153.34, 153.29, 148.94, 140.73, 134.78, 131.58, 128.61, 124.22, 120.36, 119.97, 118.23, 118.02, 117.39, 116.63, 108.36, 104,39, 97.16, 78.03, 40.68, 25.67, 25.60, 22.70, 17.53 and 15.97; and
e) fraction 'MK03' appears to be a pure nitrogenous compound.

One more embodiment of the invention relates to a composition, wherein the bioactive fraction namely MK04 obtained from the leaves of *Murrya Koenigi* for blocking of 5 lipooxygenase activity leading to the inhibition of leukotriene synthesis, suppression of interleukin-4 production having the following physicochemical characteristics:
a. Dark colored gummy material soluble in dimethyl sulfoxide;
b. TLC of bioactive material shows single spot having Rf 0.38 in the solvent system of chloroform and methanol (19:1);
c. The HPLC analysis of the bioactive material using intersil ODS-3 analytical column, solvent system methanol and a flow rate 1.0 ml/min, detection at 254 nm shows one peak with retention time 5:69 min;
d. NMR (300 MH₂, CDCl₃)δ 0.94, 1.30, 1.60, 2.04-2.10, 2.27-2.34, 2.78-2.82, 3.53-3.81, 3.85-3.92, 4.00, 4.24-4.26, and 5.26-5.41 ; and
e. the fraction MK04 is a glycolipid

One more embodiment of the invention relates to a composition, wherein the bioactive fraction namely MK05 obtained from the leaves of *Murrya Koenigi* for blocking of 5 lipooxygenase activity leading to the inhibition of leukotriene synthesis, suppression of interleukin -4 production having the following physicochemical characteristics:
a. Dark colored solid soluble in DMSO and water;
b. TLC shows single spot having Rf 0.66 in the solvent system n-butanol-acetic acid-water (9:5:7);
c. The HPLC analysis of this fraction shows a peak at retention time 21 min., solvent system methanol-water (1:9), flow rate 0.5 ml/min. and detection at 217 nm;
d. ¹³CNMR, ppm (125 MH₂, D₂O) 175.82, 169.22, 159.00, 147.63, 147.02, 144.76, 135.72, 131.28, 131.10, 129.90, 129.63, 129.20, 128.20, 127.59, 123.30, 116.81, 116.39, 115.80, 114.79, 81.77, 76.26, 76.15, 74.20, 73.79, 73.64, 73.49, 72.84, 72.16, 71.34, 70.29, 69.89, 68.83, 67.96, 63.71, 63.14, 58.22, 56.62, 56.19, 54.47, 54.42, 54.37, 41.90, 36.87 and 20.93 ; and
e. fraction MK05 appears to be aromatic compound conjugated with sugars.

One more embodiment of the invention relates to a composition, wherein the bioactive fraction, namely JB01A obtained from the leaves of Piper betle for enhancement of gamma interferon release having the following physicochemical characteristics:
a. White color solid material and is soluble in both DMSO and water;
b. TLC shows single spot having Rf 0.07 in the solvent system n-Butanol-acetic acid-water (9:5:7);
c. HPLC shows a peak at retention time 8.4 min. (JB01A, peak-1) with solvent system water-methanol (1:9), flow rate 0.5 ml/min, detection at 217 nm and intersil ODS-3 analytical column;
d. ¹³CNMR, ppm (125 MH₂, D₂O): 109.71, 108.417, 107.94, 104.17, 100.27, 84.38, 81.79, 81.53, 80.74, 77.03, 75.58, 73.85, 73.42, 71.71, 71.27, 70.74, 70.38, 69.12, 61.71, 61.00 and 60.54 ; and
e. fraction JB01A is oligosaccharide.

One more embodiment of the invention relates to a composition, wherein the bioactive fraction namely JB01B obtained from the leaves of Piper betle for enhancement of gamma interferon release having the following physicochemical characteristics:
a. White color solid soluble in both DMSO and water;
b. TLC shows single spot having Rf 0.27 in the solvent system n-butanol-acetic acid-water (9:5:7);
c. HPLC shows a peak, at retention time 8.8 min. (JB01B, peak-2) with the same above column condition;
d. ¹³CNMR, ppm (125 MH₂, D₂O): 169.48, 104.30, 98.86, 98.55, 92.60, 81.82, 76.98, 76.07, 74.57, 73.52, 73.23, 71.78, 71.46, 70.02, 69.84, 69.74, 69.30, 68.92, 68.77, 67.01, 66.43, 62.95, 62.02, 61.61, 48.67, 44.62, 38.88 ; and
e. fraction JB01 B an oligosaccharide derivative.

One more embodiment of the invention relates to a composition, wherein the bioactive fraction namely JB01C obtained from the leaves of piper betle for enhancement of gamma interferon release having the following physicochemical characteristics:
a. white solid soluble in both DMSO and water.;
b. TLC shows single spot having Rf 0.34 in solvent system n-butanol-acetic acid-water (9:5:7);
c. HPLC shows single peak at retention time 9.8 min. (JB01C, peak-3) with the same above condition;
d. NMR (300 MH₂, D₂O). 2.34. 3.27, 3.28, 3.44-3.47, 3.51, 3.60-3.63, 3.68, 3.92-3.99, 4.08, 4.92 and 5.36 ; and
e. fraction JB01C is a oligosaccharide.

One more embodiment of the invention related to a method of treating a subject for bronchial respiratory conditions said method comprising administering to the subject, an effective amount of a pharmaceutical composition comprising of lyophilized aqueous or alcoholic extract obtained from the leaves of *Piper betle (PB)* and *Murrya koenigii (M.K)* or bioactive fractions derived from these extracts, and optionally in combination with one or more pharmaceutically acceptable ingredients.

Another embodiment, the said composition is administered through oral, intravenous, intramuscular, inhalation or subcutaneous routes.

Still another embodiment, the oral route is in the form of capsule, syrup, concentrate, powder or granules.

In another embodiment of the invention provides an pharmaceutical composition useful as leukotriene and IL4 synthesis inhibitor and as Th1 type immunomudulator, said composition comprising effective amount of one or more bioactive fractions obtained from extracts of plant parts of *Piper betle (PB)* named as J801A, JBO1B and JB01C and bioactive fraction obtained from the leaf extracts of *Murrya Koenigii (M*.*K)* named as MK03, MK04 and MK05 optionally associated with or in combination with pharmaceutically acceptable additives.

One more embodiment of the invention relates to a method of treating a subject for bronchial respiratory conditions, said method comprising administering to the subject effective amount of effective amount of one or more bioactive fractions obtained from extracts of leaves of *Piper betle (PB)* named as JB01A, JBO1B and JB01C and bioactive fractions obtained from the leaf extracts of *Murrya Koenigii (M*.*K)* named as MK03, MK04 and MK05 optionally associated with or in combination with pharmaceutically acceptable additives..

In an another embodiment of the present invention, the cells in an ex-vivo human blood system are activated with calcium inophor or the likes.

In an another embodiment of the present invention, the bioactive fractions are separated from leaves of *M*. *koenigii* and *P*. *betle* by the technique such as solvent fractionation, TLC, HPTLC, HPLC etc.

In an another embodiment of the present invention, plant material are used for extraction with appropriate solvent such as methanol or water or buffer in a percolator or the equipment known in the art.

In an another embodiment of the present invention, the extracts of *M*. *koenigii* and *P. betle* leaves are concentrated under reduced pressure to save active principle.

In yet another embodiment of the present invention, the concentrates are lyophilized to remove reduced water and other residual solvent.

In yet another embodiment of the present invention, the lyophilized solid is chromatgarphed using silicagel or Sephadex LH-20 to isolate the pure fractions.

In yet another embodiment of the present invention, the isolated fractions of *M*.*K* and P.B found active as an inhibitor of 5-lipoxygenase mediated Arachidonic acid oxidation in an ex-vivo whole human blood.

In yet another embodiment of the present invention, the bioactive fractions are selected from the extracts of *M*. *koenigii* and *P. betle* inhibited 5-lipoxygenase mediated Arachidonic acid oxidation in neutrophils enriched fraction from whole blood.

In yet another embodiment of the present invention, the bioactive fractions are selected from the extracts of *M*. *koenigii* and *P. belle* are found to enhance IFN-gamma response in *ex- vivo* whole human blood.

In yet another embodiment of the present invention, the bioactive fractions selected from *M*. *koenigii* and *P. betle* extracts found to enhance INF-gamma response in *ex vivo* human blood neutrophils (PMN).

In yet another embodiment of the present invention, the bioactive fractions selected from the extracts of *M*. *koenigii* and *P. betle* found to reduce IL-4 response in human peripheral blood mononuclear cells.

In yet another embodiment of the present invention, the fractions obtained from the extracts of *M. koenigii* and *P. betle* are found as inhibitors of 5-lipoxygenase mediated arachidonic acid oxidation assayed after incubation of whole blood and lysing the cells at the time of assay are chosen for preparing the composition.

In yet another embodiment of the present invention, the lyophilized solid is chromatographed over silica gel or Sephadex LH-20 to isolate the pure bioactive fraction present in the leaf of *Murraya koenigii* and *Piper betle* for the use of relief, treatment and cure of respiratory problem.

The following examples are given by way of illustration.

### Example I

### Collection of Plant material

The leaves of plants of *Murraya Koenigii* and *Piper betle* are collected from shrubs and climber respectively from different areas of West Bengal, India. A voucher specimen is deposited at the department of Medicinal Chemistry at the Indian Institute of Chemical Biology, 4 Raja S.C. Mullick Road, Calcutta-700032.

### Example II

### Preparation of the bioactive material

### Part I (Murraya koenigii)

Fresh leaves of *Murraya koenigii* collected, cleaned and washed with water after getting from the local supplier and used as starting materials.

410 gm of the fresh leaves of *Murraya koenigii* is made a paste in a mixture - blender in a methanol (1000 ml) and is placed in a glass percolator (5 lit. capacity) with the addition of 1000 ml of methanol. This is kept for 16 hrs (overnight) at room temperature. Filtering the extract through Whatman No.1 tilter paper collected the percolate. The process of extraction is repeated four times and the combined extract is evaporated to dryness under reduced pressure in a rotary evaporator, keeping the temperature at 40°C (bath). The solid residual matter left is viscous in appearance and it is further dried by lyophilization. The yield is 25.63 gm (MKOC). The schematic diagram of the fractionation procedure is shown in flow diagram 1.

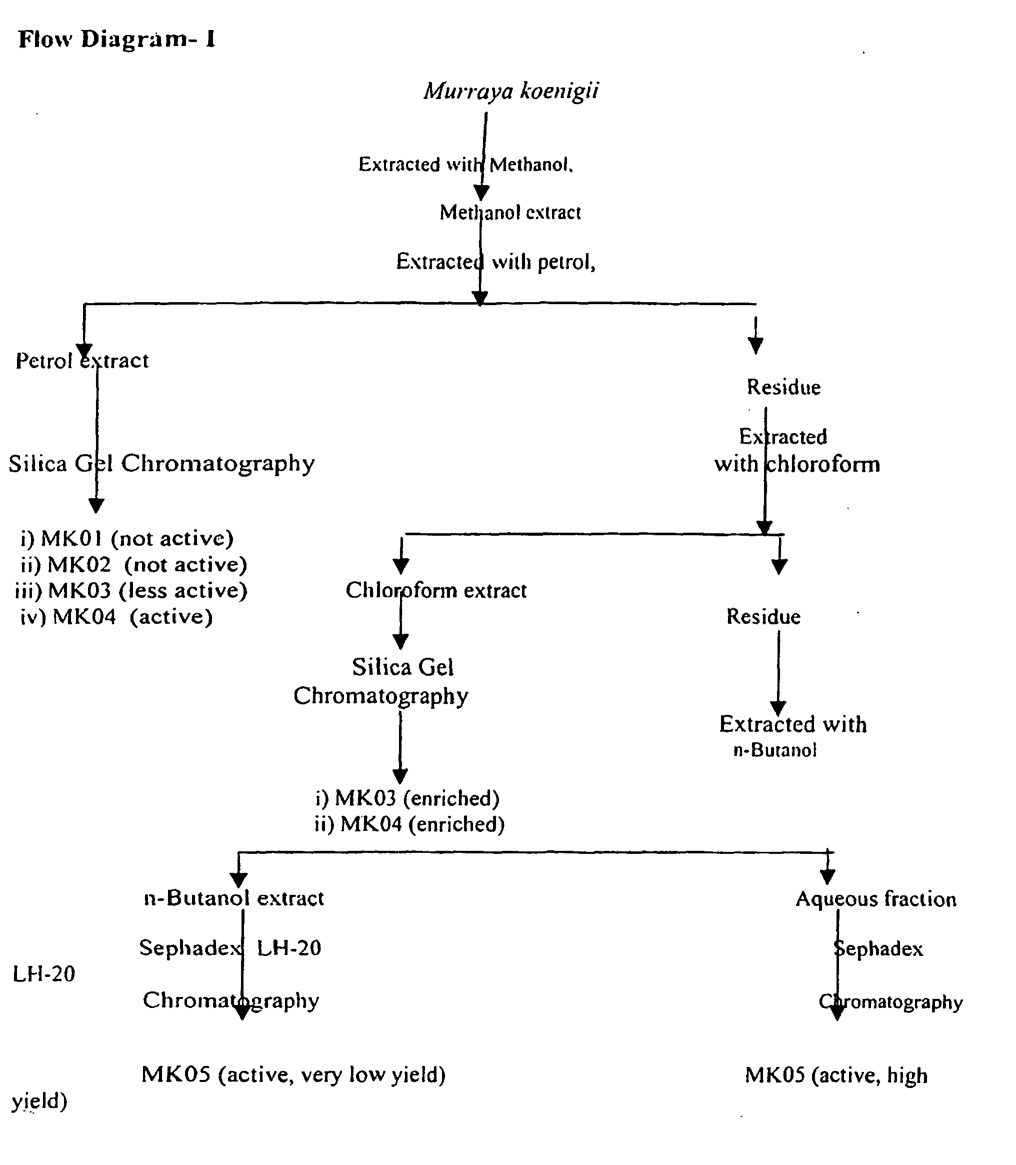

### Example 3

150 gm of fresh leaves of *M*. *koenigii* thoroughly washed in sterile water was homogenized with 750 ml of glass distilled water in a mixture-blender and then sonicated in an ultrasonic bath with 5 burst each for 5 minutes. Filtering through Whatman no 1 filter paper separated the material extracted in water. This type of treatment for extracted was repeated for three times. The combines extract was byophilized yielding a powdered material (MKOC) 11 gm in weight.

### Example 4 Part II (P. betle) extract

Fresh leaves of *Piper betle* is collected from local supplier and is used as starting material.

The fresh leaves of P betle (1.5kg homogenized with distilled water (500ml)in a mixture-blender and then sonicated in an ultrasonic bath with 3 burst each for 15 min. It was allowed to be extracted overnight or 16 hrs. Filtering through Whatman No.1 filter paper separated the material extracted in water. This type of treatment for extraction was repeated for three times. The combined extract was evaporated to dry in a flash evaporator under reduced pressure at 40°C. The residual substance was then dried in a desiccator under high vacuum and the semi-solid (14.92 gm) (JB01) was left. The schematic diagram of the fractionation procedure is shown in flow diagram II.

### Example 5 (Extract of M. koenigii and extract of P. betle)

3 gm extract of *M. koenigii* and 3 gm extract of *P. belle* were mixed together homogeneously with adding a few drops of water. The whole extract was evaporated to dryness in a freeze-drier. This extract was tested for biological activity (MKOC+JB01).

### Example 6 (MK+PB)

200 g of each plant parts is homogenized separately with 300 ml of methanol in a mixture-blender. It is then sonicated in an ultrasonic bath with 3 burst each for 15 min. The extract is filtered through Whatman No.1 filter paper and the filtrate is collected. This process of extraction is repeated three times. The combined extract is lyophilized yielding a semi-solid man weighing 6.32 g

### Example 7

Fresh leaves of *M*. *koenigii* (70 gm) and *P. betle* (500 gm) were mixed together and was thoroughly washed with distilled water. 1 It was homogenized with distilled water (300 ml) in a mixture-blender. It was then sonicated in an ultrasonic bath with 3 burst each for 15 min. The extract was filtered through Whatman No.1 filter paper and the filtrate was collected. This process of extraction was repeated three times. The combined extract was lyophilized yielding a semi-solid mass (10 gm). This was then tested for biological activity (MKOc+JB01)
Since the leaf of both the plants are widely used for making various food preparations in India, its biocompatibility and toxicity need not worry anyone. Further, both the plants are ubiquitous. The presence of the bioactive factors in the leaf of *Murraya koenigii* and *Piper betle* useful for relief, treatment and cure of respiratory problems has been demonstrated here and its preparation is fast, convenient and inexpensive. Feeding of leaf extract to animals did not produce any adverse effect.

A part of this extract (4.00 gm) is chromatographed, over sephadex LH-20 column. Among the isolated fractions, three designated as JB01A (0.17g or 70 mg,) JB01B (0.33 g or 330 mg) and JB01C (0.94 g or 940 mg) are found active.

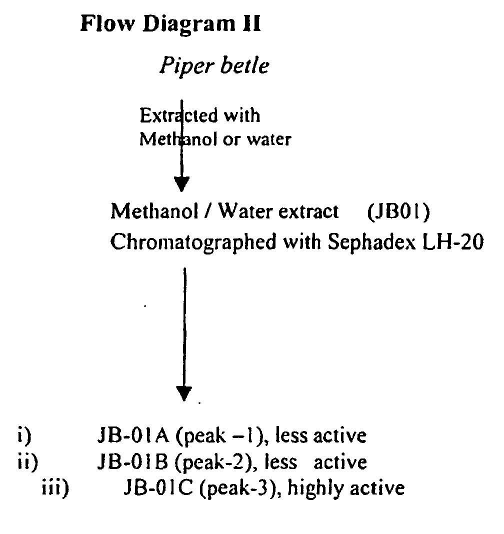

### Example 8

The method of preparation of the bioactive factors comprises:
1. collecting the fresh leaves and all other plant parts from the local suppliers
2. drying the plant parts under shade to a moderate degree or to take the fresh plant parts as the starting material
3. powdering the dried or homogenizing each plant parts separately in the apparatus known in the art.
4. putting the powder or homogenate in a percolator under the bulk of appropriate solvents; choosing hydrocarbon solvents such as petroleum ether (B.P.40 - 60°C), petroleum ether (B.P. - 60°C - 80°C), pentane, hexane, benzene etc., chlorinated solvents like chloroform, dichloromethane, carbon tetrachloride etc., etherial solvents such as diethyl ether, tetrahydrofuran, dioxane etc., ketonic solvents such as acetone, cyclo pentanone etc; ester solvents such as ethyl acetate, ethyl formate etc.; all alcohols such as methanol, ethanol, n-butanol etc., water and buffers.
5. extracting the percolated plant material using a percolator or the apparatus or equipment currently known in the art over a period of time ranging from 1 to 120 hours.
6. Evaporating the solvent under reduced pressure using an apparatus or equipment currently known in the art.
7. Lyophilizing or drying the material in the apparatus or equipment currently known in the art and storing the processed material in a cool and dry place in an air-tight container.
8. isolation of individual bioactive fractions by silica gel and Sephadex LH-20 chromatography.
9. Characterizations of pure compounds present in the extract to be used as markers for evaluating the bioactivity.
10. Storing the processed material in a cool and dry place in an airtight container.
11. Evaluating the bioactivity of the material

### Example 9

Description of the method for measuring inhibition of Arachidonic acid oxidation. 500 µl of heparinised human peripheral blood half diluted with PBS was taken in each well of a 24-well tissue culture plate. 10-µg/ml concentration of each extract sample was added in each well. The cells were then incubated for 3 hr. at 37°C with occasional shaking. The 10µl/ml of arachidonic acid soloution (12.2 mg/ml of absolute alcohol stored under argon at -20°C) was added to each well for 5 min prior to the addition of calcium ionophore (A23187) at a concentration of 20µg/ml to continue the incubation for further 10 min. Volume of the cell suspension was made upto 2 ml with PBS and its oxygen content was monitored with the help of a sensitive Oxygraph instrument.

### Example 10

### Method:

500 µl of heparinised human peripheral blood half diluted with PBS is taken in each well of a 24-well tissue culture plate. 1µg/ml concentration of each sample is added in each well. In case of mixture, the fractions are mixed at 1:1 ratio to make a final preparation, which is used at a concentration of 1.0 µg/ml. The cells are then incubated for 3 hr. at 37°C with occasional shaking. The 10µl/ml of arachidonic acid solution (12.2 mg/ml of absolute alcohol stored under argon at -20°C) is added to each well for 5 min prior to the addition of calcium ionophore (A23187) at a concentration of 20µg/ml to continue the incubation for further 10 min. Volume of the cell suspension is made up to 2 ml with PBS and its oxygen content is monitored with the help of a sensitive Oxygraph instrument.

### Example-11

500 µl of half diluted human peripheral blood was incubated with 10 µg/ml of each extract for 3 hr. at 37°C with occasional shaking. Then 10µl/ml of arachidonic acid solution (12.2 mg/ml of absolute alcohol stored under argon at -20°C) was added to each well for 5 min prior to the addition of calcium ionophore (A23187) at a concentration of 20µg/ml to continue the incubation for further 10min. Volume of the cell suspension was made upto 2 ml by addition 1 ml of PBS+500 µl of water to lyse the cells. Oxygen content of the cell lysate was monitored in a sensitive Oxygraph instrument.

### Example- 12

### Method:

500 µl of half diluted human peripheral blood is incubated with 1 µg/ml of each fraction for 3 hr. at 37°C with occasional shaking. In case of mixture, the fractions are mixed at 1:1 ratio to make a final preparation, which is used at a concentration of 1.0 µg/ml. Then 10µl/ml of arachidonic acid solution (12.2 mg/ml of absolute alcohol stored under argon at -20°C) is added to each well for 5 min prior to the addition of calcium ionophore (A23187) at a concentration of 20µg/ml to continue the incubation for further 10 min. Volume of the cell suspension is made up to 2 ml by addition 1 ml of PBS+500 µl of water to lyse the cells. Oxygen content of the cell lysate is monitored in a sensitive Oxygraph instrument.

### Example - 13

Heparinized human peripheral blood was mixed with equal volume of 2% gelatine solution in PBS and allowed to stand for half an hour when RBC were settled down at the bottom. Upper layer containing neutrophil enriched mononuclear cells was centrifuged and cell pellet was suspended in PBS. 500µl of this cell suspension (3X10⁶ cells/well) was incubated with 10µg/ml of each sample at 37°C for 3 hrs. Then arachidonic acid solution 10µl/ml (12.2 mg/ml) was added to each well for 5 min-prior to the addition of calcium ionophore (A23187) at a concentration of 20µg/ml to continue the incubation for further 10 min. Volume of the cell was made upto 2 ml with PBS and oxygen content of the cell suspension was monitored in a sensitive Oxygraph instrument.
Thus, extracts of *M. koenigii* and *P. betle*, have been screened for biological activity relevant to the relief, treatment and cure of respiratory problems, and the processed extract, have been analysed by Thin Layer chromatography and HTPLC. The blended extract was dissolved and suspended finely in appropriate solvents and used in the test system (ex vivo whole human blood to establish the stipulated biological response.

### Example 14

### Method

Heparinised human peripheral blood is mixed with equal volume of 2% gelatin solution in PBS and allowed to stand for half an hour when RBC are settled down at the bottom. Upper layer containing neutrophil enriched mononuclear cells is centrifuged and cell pellet is suspended in PBS. 500µl of this cell suspension (3X10⁶ cells/well) is incubated with 1 µg/ml of each fraction at 37°C for 3 hrs. In case of mixture, the fractions are mixed at 1:1 ratio to make a final preparation, which is used at a concentration of 1.0 µg/ml. Then arachidonic acid solution 10µl/ml (12.2 mg/ml) is added to each well for 5 min prior to the addition of calcium ionophore (A23187) at a concentration of 20µg/ml to continue the incubation for further 10 min. Volume of the cell is made upto 2 ml with PBS and oxygen content of the cell suspension is monitored in a sensitive Oxygraph instrument.

Thus, bioactive fractions from the extracts of the leaves of *M. koenigii* and *P. betle,* have been screened for biological activity relevant to the relief, treatment and cure of asthmatic conditions, and'the fraction thus processed, have been analyzed by Thin Layer chromatography and HPLC and NMR spectra, dissolved and suspended finely in appropriate solvents and used in the test system (*ex vivo* whole human blood to establish the stipulated biological response.

### Example-15

### Preparation of intracellular interferon gamma (IFN - γ) and interleukin -4 (IL-4) by flow cytometry

Heparinized whole blood (0.1 ml/ well of 24 well plates,collected from normal individuals) were cultured at 37° C in 5% CO₂ for 6 hours in a total volume of 1.0 ml Rosewell Park Memorial Institute (RPMI) medium containing 10% heat inactivated fetal bovine serum and phytohaemaglutinin( PHA, 10 µg/ml) in the presence or absence of *P.betle* and *M*.*koenigii* leaves extract (1.0 mg / ml each) either alone or in combination. To cause the intracellular accumulation of newly synthesized proteins, brefeldin A (10 µg/ml) was added to the cells for last 4 hours. At the end of incubation period, cells were treated with FACS™ lysing solution (Becton Dickinson, USA) for lysis of RBC. Cells were then washed, permeabilized by treatment with 4% paraformaldehyde for 10 minutes. After washing with washing buffer (phosphate buffered saline [PBS] containing 1% albumin, 0.1% saponin and 0.1% Sodium azide), permeabilized cells were treated with either FITC-labeled anti-IFN γ monoclonal antibody or PE-labeled anti-IL-4 monoclonal antibody for 20 minutes in room temperature at dark. Cells were washed with washing buffer and then resuspended in PBS containing 1% paraformaldehyde for single colour flow cytometry analysis using FACS Calibur (Becton Dickinson, USA) with the programme Cell Quest. Ten thousand cells were collected for each sample and the fluorescence intensity was measured on a logarithmic scale. To make sure that only intracellular IFN γ or IL- 4 was being detected, cells were stained with FITC- labeled anti-IFN γ or PE -labeled anti-IL-4 antibody before permeabilization and gave less than 0.2% fluorescent cells for each staining. Irrelevant isotype-matched control antibody also produced only less than 0.1% fluorescent cells.
Flow cytometric determination of intracellular IFN-gamma and IL-4 in ex vivo human mononuclear cells after co-culture with phytohemmaglutinin in the presence or absence of extracts obtained from *M. Koenigii* and *P*.*betle*. Our data indicate that the extract from P. betle increased IFN-gamma positive cells and reduced IL-4 positive cells markedly. M. *koenigii* extract had reduced IL-4 positive as well as IFN-gamma positive cells. Combination of these two extracts increased IFN-gamma positive cells to the control level and still maintained reduced level of IL-4 positive.

### Example 16

A part of *M*. *koengii* extract (8.01g) is subjected to silica gel column chromatography that resulted in isolation of fractions among the biologically active fractions. These are designated as fraction MK03 (70 mg), MK04 (270 mg) and MK05 (110 mg).

### Example 17

### Physicochemical Characterization of MK03, MK04, MK05, JB01A, JB01B and JB01C

### Fraction MK03:

1. The dried solid, melting point 98 - 100°C, soluble in DMSO.
2. Thin layer chromatography shows single spot having Rf 0.48 in one of the solvent systems - chloroform and methanol (19:1)
3. The HPLC analysis shows single peak with retention time 8.5 min. at a flow rate of 0.5 ml/min using intersel ODS-3 (4.6 x 250 mm) analytical column, solvent system methanol and detection is carried out at 210 nm. HPLC: Column ODS-3 (4.6 x 250 mn) Flow rate - 0.5 m/min; Peak at 210 nm; Retention time - 3.5 min.; Solvent system - methanol.
4. ¹³CNMR, ppm (125 MH₂, CDCl₃): 153.34, 153.29, 148.94, 140.73, 134.78, 131.58, 128.61, 124.22, 120.36, 119.97, 118.23, 118.02, 117.39, 116.63, 108.36, 104.39, 97.16, 78.03, 40.68, 25.67, 25.60, 22.70, 17.53 and 15.97
5. Fraction 'MK03' appears to be a pure alkaloid

### Fraction MK04:

1. Dark colored gummy material soluble in dimethyl sulfoxide.
2. TLC of bioactive material shows single spot having Rf 0.38 in the solvent system of chloroform and methanol (19:1)
3. The HPLC analysis of the bioactive material using intersil ODS-3 analytical column, solvent system methanol and a flow rate 1.0 ml/min, detection at 254 nm shows one peak with retention time 5:69 min.
4. NMR (300 MH₂, CDCl₃)δ 0.94, 1.30, 1.60, 2.04 - 2.10, 2.27 - 2.34, 2.78-2.82, 3.53 - 3.81, 3.85 - 3.92, 4.00, 4.24 - 4.26, and 5.26 - 5.41
5. The fraction MK04 is a glycolipid

### Fraction MK05:

1. Dark colored solid soluble in DMSO and water.
2. TLC shows single spot having Rf 0.66 in the solvent system n-butanol-acetic acid - water (9:5:7)
3. The HPLC analysis of this fraction shows a peak at retention time 21 min., solvent system methanol - water (1:9), flow rate 0.5 ml/min. and detection at 217 nm.
4. ¹³CNMR, ppm (125 MH₂, D₂O): 175.82, 169.22, 159.00, 147.63, 147.02, 144.76, 135.72, 131.28, 131.10, 129.90, 129.63, 129.20, 128.20, 127.59, 123.30, 116.81, 116.39, 115.80, 114.79, 81.77, 76.26, 76.15, 74.20, 73.79, 73.64, 73.49, 72.84, 72.16, 71.34, 70.29, 69.89, 68.83, 67.96, 63.71, 63.14, 58.22, 56.62, 56.19, 54.47, 54.42, 54.37, 41.90, 36.87 and 20.93
5. The fraction MK05 appears to be aromatic compound conjugated with sugars.

### Fraction JB01A:

1. White color solid material and soluble in both DMSO and water.
2. TLC shows single spot having Rf 0.07 in the solvent system n-Butanol-acetic acid - water (9:5:7)
3. HPLC shows a peak at retention time 8.4 min. (JB01A, peak-1) with solvent system water-methanol (9:1), flow rate 0.5 ml/min, detection at 217 nm and intersil ODS-3 analytical column.
4. ¹³CNMR, ppm (125 MH₂, D₂O): 109.71, 108.417, 107.94, 104.17, 100.27, 84.38, 81.79, 81.53, 80.74, 77.03, 75.58, 73.85, 73.42, 71.71, 71.27, 70.74, 70.38, 69.12, 61.71, 61.00 and 60.54
5. Fraction JB01A is oligosaccharide

### Fraction JB01B:

1. White color solid soluble in both DMSO and water
2. TLC shows single spot having Rf 0.27 in the solvent system n-butanol-acetic acid - water (9:5:7)
3. HPLC shows a peak, at retention time at 8.8 min (JB01B, peak-2) with the same above column condition
4. ¹³CNMR, ppm (125 MH₂, D₂O): 169.48, 104.30, 98.86, 98.55, 92.60, 81.82, 76.98, 76.07, 74.57, 73.52, 73.23, 71.78, 71.46, 70.02, 69.84, 69.74, 69.30, 68.92, 68.77, 67.01, 66.43, 62.95, 62.02, 61.61, 48.67, 44.62, 38.88.
5. Fraction is JB01B an oligosaccharide derivative.

### Fraction JB01C:

1. White solid soluble in both DMSO and water.
2. TLC shows single spot having Rf 0.34 in solvent system n-butanol - acetic acid-water (9:5:7).
3. HPLC shows single peak at retention time 9.8 min.(JB01C, peak-3) with the same above condition.
4. NMR (300 MH₂, D₂O). 2.34. 3.27, 3.28, 3.44 - 3.47, 3.51, 3.60 - 3.63, 3.68, 3.92 - 3.99, 4.08, 4.92 and 5.36.
5. Fraction JB01C is an oligosaccharide.

### Example 18

### Description of test for analyzing intracellular interferon gamma (IFN-γ) and interleukin -4 (IL-4) by flowcytometry

Method: Heparinized whole blood (0.1 ml/ well of 24 well plates, collected from normal individuals) are cultured at 37° C in 5% CO₂ for 6 hours in a total volume of 1.0 ml Rosewcll Park Memorial Institute (RPMI) medium containing 10% heat inactivated fetal bovine serum and phorbol myristate acetate (PMA) and inomycin or LPS in the presence or absence of fractions from all plant parts of *P. betle* and *M*. *koenigii* (50.0 µg / ml each) either alone or in combination. To cause the intracellular accumulation of newly synthesized proteins, brefeldin A (10 µg/ml) is added to the cells for last 4 hours. At the end of incubation period, cells are treated with FACS™ lysing solution (Becton Dickinson, USA) for lysis of RBC. Cells are then washed, permeabilized by treatment with 4% paraformaldehyde for 10 minutes. After washing with washing buffer (phosphate buffered saline [PBS] containing 1% albumin, 0.1% saponin and 0.1% sodium azide), permeabilized cells are treated with either FITC-labeled anti-IFN-γ monoclonal antibody or PE-labeled anti-IL-4 monoclonal antibody for 20 minutes in room temperature at dark. Cells are washed with washing buffer and then re-suspended in PBS containing 1% paraformaldehyde for single color flow cytometric analysis using FACS Calibur (Becton Dickinson, USA) with the programme Cell Quest. Ten thousand cells are collected for each sample and the fluorescence intensity is measured on a logarithmic scale. To make sure that only intracellular IFN γ or IL- 4 is being detected, cells are stained with FITC-labeled anti-IFN γ or PE -labeled anti-IL-4 antibody before permealization and gave less than 0.2% fluorescent cells for each staining. Irrelevant isotype-matched control antibody also produced only less than 0.1% fluorescent cells.

### Results of Example -9

| Fractions | % inhibition of O₂ consumption |
|---|---|
| None | -- |
| MK0C | 81.1 |
| JB01 | 3 |
| MK0C+JB01 | 77.73 |

### Results - Example -11

| Fractions | % inhibition of O₂ consumption |
|---|---|
| None | -- |
| MK0C | 89.62 |
| JB01 | 5.42 |
| MK0C+JB01 | 83.20 |

### Results of Example-13

| Fractions | % inhibition of O₂ consumption |
|---|---|
| None | -- |
| MK0C | 88.76 |
| JB01 | 4.58 |
| MK0C+JB01 | 85.94 |

**Table 1**

| **showing results of examples 10, 12 & 14** | | | |
|---|---|---|---|
| Bioactive Fractions | % inhibition of O₂ consumption | | |
| | Example 10 | Example 12 | Example 14 |
| None | - | - | - |
| MK03 | 12.75 | 10.16 | 11.68 |
| MK04 | 86.42 | 76.57 | 87.40 |
| MK05 | 80.45 | 79.70 | 89.84 |
| JB01C | 3 | 5.42 | 4.58 |
| MK03+MK04+MK05 | 81 | 89.62 | 88.76 |
| MK03+MK04+MK05+JB01C | 77.73 | 83.20 | 85.94 |

### Example 19

Results of therapeutic evaluation of the preparation developed by combination of M *koenigii* bark and P. betle leaves extract for the treatment of respiratory trouble. (Conducted by a Registered Auyrvedic practitioner)

| Patient | Sex / Age | Nature of respiratory | Duration of disease | Treatment period | Improvement |
|---|---|---|---|---|---|
| A | Male / 46 | Recurrent wheezing, difficult breathing at nighttime, chest tightness, cough worsening at night, domestic dust allergy and breathing problem. Allergic to fur / smoking | 27 years | 8 weeks | 90% All the inhaler (6 puffs) were withdrawn |
| B | Male / 39 | Recurrent wheezing, occasional chest tightness. difficult in breathing. | 14 years | 8 weeks | 80 % No breathing problem residual shortening occasionally |
| C | Male / 62 | Recurrent wheezing, difficult breathing at nighttime, occasional chest tightness, dust allergy causing breathing problem. Exercise induced breathing difficulties. | 50 years | 7 weeks | 70% No wheezing, No exercise induced breathing problem. No chest tightness |
| D | Female / 28 | Cough, worse particularly at night, recurrent wheezing, recurrent difficult breathing, recurrent chest tightness, viral infection, allergy to domestic dust mites and change in temperature | 20 years | 8 weeks | 90% no inhaler requirement and no other drug is necessary |
| E | Male / 87 | Cough, worse particularly at night, recurrent wheezing, recurrent difficult breathing, recurrent chest tightness, viral infection, allergy to domestic dust mites and change in temperature | 30 years | 8 weeks | 90 % No inhaler is currently required. No drug requirement. Bed ridden patient is moving on wheel chair without any assistance. |

## Claims

1. A synergistic pharmaceutical composition useful as leukotrine inhibitor, IL4 synthesis inhibitor and Th1 type immunomodulator, the said composition comprising effective amounts of lyophilized, aqueous or alcoholic extracts obtained from the leaves of *Piper betle (PB)* and *Murrya koenigii (M.k)* or its bioactive fractions JB01A, JB01B, JB01C and MK03, MK04, MK05 respectively in all possible combinations, derived from these extracts with optionally one or more pharmaceutically acceptable ingredients, wherein the ratio of *PB* extract or bioactive fractions derived from this extract extract to *M.k* extract or bioactive fractions derived from this extract is in the range of 1: 1 to 1:5.

2. The pharmaceutical composition as claimed in claim 1, wherein
the bioactive fraction named MK03 has the following physicochemical characteristics:
(a) the dried solid, melting point 98-100°C soluble in DMSO;
(b) Thin layer chromatography shows single spot having Rf 0.48 in one of the solvent systems-chloroform and methanol (19:1);
(c) The HPLC analysis shows single peak with retention time 8.5 min. at a flow rate of 0.5 ml/min using intersel ODS-3 (4.6 x 250 mm) analytical column, solvent system methanol and detection is carried out at 210 nm. HPLC: Column ODS-3 (4.6 x 250 mm) Flow rate - 0.5 ml/min; Peak at 210 nm; Retention time-3.5 min.; Solvent system - methanol
(d) ¹³CNMR, ppm (125 MH₂, CDCl₃): 153.34, 153.29, 148.94, 140.73, 134.78, 131.58, 128.61, 124.22, 120.36, 119.97, 118.23, 118.02, 117.39, 116.63, 108.36, 104,39, 97.16, 78.03, 40.68, 25.67, 25.60, 22.70, 17.53 and 15.97; and
(e) fraction 'MK03' appears to be a pure nitrogenous compound;
the bioactive fraction named MK04 has the following physicochemical characteristics:
(a) Dark·colored gummy material soluble in dimethyl sulfoxide;
(b) TLC of bioactive material shows single spot having Rf 0.38 in the solvent system of chloroform and methanol (19:1);
(c) The HPLC analysis of the bioactive material using intersil ODS-3 analytical column, solvent system methanol and a flow rate 1.0 ml/min, detection at 254 nm shows one peak with retention time 5:69 min;
(d) NMR (300 MH₂, CDCl₃)δ 0.94, 1.30, 1.60, 2.04-2.10, 2.27-2.34, 2.78-2.82, 3.53-3.81, 3.85-3.92, 4.00, 4.24-4.26, and 5.26-5.41 ; and
(e) the fraction MK04 is a glycolipid
and the bioactive fraction named MK05 has the following physicochemical characteristics:
(a) Dark colored solid soluble in DMSO and water;
(b) TLC shows single spot having Rf 0.66 in the solvent system n-butanol-acetic acid-water (9:5:7);
(c) The HPLC analysis of this fraction shows a peak at retention time 21 min., solvent system methanol-water (1:9), flow rate 0.5 ml/min. and detection at 217 nm;
(d) ¹³CNMR, ppm (125 MH₂, D₂O): 175.82, 169.22, 159.00, 147.63, 147.02, 144.76, 135.72, 131.28, 131.10, 129.90, 129.63, 129.20, 128.20, 127.59, 123.30, 116.81, 116.39, 115.80, 114.79, 81.77, 76.26, 76.15, 74.20, 73.79, 73.64, 73.49, 72.84, 72.16, 71.34, 70.29, 69.89, 68.83, 67.96, 63.71, 63.14, 58.22, 56.62, 56.19, 54.47,54.42, 54.37, 41.90, 36.87 and 20.93; and
(e) fraction MK05 appears to be aromatic compound conjugated with sugars.

3. The pharmaceutical composition as claimed in claim 1, wherein the bioactive fraction JB01A has the following physicochemical characteristics:
(a) White color solid material and is soluble in both DMSO and water;
(b) TLC shows single spot having Rf 0.07 in the solvent system n-Butanol-acetic acid-water (9:5:7);
(c) HPLC shows a peak at retention time 8.4 min. (JB01A, peak-1) with solvent system water-methanol (1:9), flow rate 0.5 ml/min, detection at 217 nm and intersil ODS-3 analytical column;
(d) ¹³CNMR, ppm (125 MH₂, D₂O): 109.71, 108.417, 107.94, 104.17, 100.27, 84.38, 81.79, 81.53, 80.74, 77.03, 75.58, 73.85, 73.42, 71.71, 71.27, 70.74, 70.38, 69.12, 61.71, 61.00 and 60.54 ; and
(e) fraction JB01A is oligosaccharide;
the bioactive fraction JB01B has the following physicochemical characteristics:
(a) White color solid soluble in both DMSO and water;
(b) TLC shows single spot having Rf 0.27 in the solvent system n-butanol-acetic acid-water (9:5:7);
(c) HPLC shows a peak, at retention time 8.8 min. (JB01B, peak-2) with the same above column condition;
(d) ¹³CNMR, ppm (125 MH₂, D₂O): 169.48, 104.30, 98.86, 98.55, 92.60, 81.82, 76.98, 76.07, 74.57, 73.52, 73.23, 71.78, 71.46, 70.02, 69.84, 69.74, 69.30, 68.92, 68.77, 67.01, 66.43, 62.95, 62.02, 61.61, 48.67, 44.62, 38.88 ; and
(e) fraction JB01B an oligosaccharide derivative;
and the bioactive fraction JB01C has the following physicochemical characteristics:
(a) white solid soluble in both DMSO and water.;
(b) TLC shows single spot having Rf 0.34 in solvent system n-butanol-acetic acid-water (9:5:7);
(c) HPLC shows single peak at retention time 9.8 min. (JB01 C, peak-3) with the same above condition;
(d) NMR (300 MH₂, D₂O). 2.34. 3.27, 3.28, 3.44-3.47, 3.51, 3.60-3.63, 3.68, 3.92-3.99, 4.08, 4.92 and 5.36 ; and
(e) fraction JB01C is a oligosaccharide.

4. The composition as claimed in claim 1, wherein the pharmaceutically acceptable ingredient used is selected from nutrients such as proteins, carbohydrates, sugar, talc, magnesium stearate, cellulose, calcium carbonate, starch-gelatin paste and/or carriers, excipients, diluents or solvents.

5. The composition as claimed in claim 1, wherein the said composition is administered through inhalation, oral, intravenous, intramuscular, subcutaneous routes or any other suitable routes.

6. The composition as claimed in claim 1, wherein the oral route is in the form of capsule, syrup, concentrate, powder or granules.

7. The composition as claimed in claim 1, wherein the amount administered by intravenous route is less than the oral route.

8. The composition as claimed in claim 1, which is administered at a dosage level between 1 to 20 mg/kg of body weight at least once in a day for a period of at least 4 weeks.

9. The composition as claimed in claim 1, wherein the composition consists of bioactive fractions MK03, MK04 and/or MK05.

10. The composition as claimed in claim 1, wherein the composition consists of bioactive / fractions JB01C, MK03, MK04 and/or MK05.

11. The composition as claimed in claim 1, wherein the amount of bioactive fractions administered by intravenous route is less than the oral route.

12. The composition as claimed in claim 1, wherein the proportion of bioactive fractions from *M*.*k* is equal to or greater than the bioactive fraction of (PB) *piper betle*.

13. The composition as claimed in claim 1, wherein the composition is administered at a dosage level between 0.5 to 10.0 mg/kg of body weight at least once in a day for a period at least 4 weeks depending upon the respiratory conditions.

14. The composition as claimed in claim 1, wherein, the composition is administered for a period for at least 4 weeks and up to 3 months.

15. The composition as claimed in claim 1, wherein the combination of bioactive fractions MK03, MK04 and MK05 obtained from the leaves and other plant parts of *Murrya Koenigi* for blocking of 5 lipooxygenase activity leads to the inhibition of leukotriene synthesis, suppression of interleukin - 4 production and as Th1 type immunomudulator

16. The composition as claimed in claim 1, wherein the combination of bioactive fractions MK03, MK04 and MK05 obtained from the leaves and other plant parts of *Murrya Koenigi (M*.*k)* and JB01C obtained from the leaves and other plant parts of piper betle for blocking of 5 lipooxygenase activity leads to the inhibition of leukotriene synthesis, suppression of interleukin - 4 production and for enhancement of gamma interferon release

17. The composition as claimed in claim 1, is used for the treatment of bronchial respiratory conditions.

18. The composition as claimed in claim 1, is used for treating mammals and animals.

19. The composition as claimed in claim 1, is used for shifting Th2 response to Th1 response.

20. The composition as claimed in claim 1, is used for inhibiting 5-tipooxygenase mediated Arachidonic acid oxidation in neutrophils enriched component of whole blood.

21. The composition as claimed in claim 1, is used for enhancing IFN-gamma and reducing IL-4 response in ex-vivo human whole blood.

22. The composition as claimed in claim 1, is used for enhancing IFN-gamma response in ex vivo human whole blood mononuclear (PMN)

23. The composition as claimed in claim 1, is used for reducing IL-4 response in human peripheral whole blood mononuclear cells.

## Patentansprüche

1. Synergistische pharmazeutische Zusammensetzung zum Einsatz als Leukotrin-Inhibitor, IL4-Synthese-Inhibitor und als Immunmodulator des Typs Th1, wobei die Zusammensetzung wirksame Mengen an lyophilisierten, wässrigen oder alkoholischen Extrakten, die aus den Blättern der Piper betle (PB) und der Murrya koenigii (M.k.) erhältlich sind, oder an bioaktiven Fraktionen JB01A, JB01B, JB01C beziehungsweise MK03, MK04, MK05, die von diesen Extrakten abgeleitet sind, in allen möglichen Kombinationen und wahlweise ein oder mehrere pharmazeutisch verträgliche Inhattsstoffe umfasst, wobei das Verhältnis des PB-Extrakts oder der bioaktiven Fraktionen, die von diesem Extrakt abgeleitet sind, zu dem M.k.-Extrakt oder zu den bioaktiven Fraktionen, die von diesem Extrakt abgeleitet sind, im Bereich 1:1 bis 1: 5 liegt.

2. Pharmazeutische Zusammensetzung nach Anspruch 1, wobei die MK03 genannte bioaktive Fraktion die folgenden physikalisch chemischen Eigenschaften aufweist:
(a) der getrocknete Feststoff weist einen Schmelzpunkt zwischen 98 und 100 °C auf und ist löslich in DMSO;
(b) die Dünnschichtchromatographie zeigt in einem der Lösungsmittelsysteme Chloroform und Methanol (19:1) einen einzigen Fleck mit einem Rf von 0,48 auf;
(c) die HPLC-Analyse zeigt unter Verwendung der analytischen Säule Intersel ODS-3 und dem Lösungsmittelsystem Methanol einen einzigen Ausschlag mit einer Verzögerungszeit von 8,5 min bei einer Flussrate von 0,5 ml/min auf, wobei der Nachweis bei 210 nm durchgeführt wurde. HPLC: Säule ODS-3 (4,6 x 250 mm), Flussrate 0,5 ml/min; Ausschlag bei 210 nm; Verzögerungszeit 3,5 min; Lösungsmittelsystem Methanol;
(d) ¹³CNMR, ppm (125 MH₂, CDCl₃): 153,34, 153,29, 148,94, 140,73, 134,78, 131,58, 128,61, 124,22, 120,36, 119,97, 118,23, 118,02, 117,39, 116,63, 108,36, 104,39, 97,16, 78,03, 40,68, 25,67, 25,60, 22,70, 17,53 und 15,97 und
(e) die Fraktion ,MK03' hat den Anschein eine reine stickstoffhaltige Verbindung zu sein,
die MK04 genannte bioaktive Fraktion weist die folgenden physikalisch chemischen Eigenschaften auf:
(a) dunkel gefärbtes gummiartiges Material, welches in Dimethylsulfoxid löslich ist;
(b) die TLC (Dünnschichtchromatographie) des bioaktiven Materials zeigt in dem Lösungsmittelsystem Chloroform und Methanol (19:1) einen einzigen Fleck mit einem Rf von 0,38 auf;
(c) die HPLC-Analyse zeigt unter Verwendung der analytischen Säule Intersil ODS-3, mit dem Lösungsmittelsystem Methanol, einer Flussrate von 1,0 ml/min und bei einem Nachweis bei 254 nm einen Ausschlag mit einer Verzögerungszeit von 5:69 min auf;
(d) NMR (300 MH₂ CDCl₃) δ 0,94, 1,30, 1,60, 2,04-2,10, 2,27-2,34, 2,78-2,82, 3,53-3,81, 3,85-3,92, 4,00, 4,24-4,26, und 5,26-5,41 und
(e) die Fraktion MK04 ist ein Glykolipid,
wobei die MK05 genannte bioaktive Fraktion die folgenden physikalisch chemischen Eigenschaften aufweist:
(a) dunkel gefärbter Feststoff, der in DMSO und in Wasser löslich ist;
(b) die TLC zeigt in dem Lösungsmittelsystem N-Butanol-Essigsäure-Wasser (9:5:7) einen einzigen Fleck mit einem Rf von 0,66 auf;
(c) die HPLC-Analyse dieser Fraktion zeigt mit dem Lösungsmittelsystem Methanol-Wasser (1:9), einer Flussrate von 0,5 ml/min und bei einem Nachweis bei 217 nm einen einzigen Ausschlag bei einer Verzögerungszeit von 21 min auf;
(d) ¹³CNMR, ppm (125 MH₂,D₂O): 175,82, 169,22, 159,00, 147,63, 147,02, 144,76, 135,72, 131,28, 131,10, 129,90, 129,63, 129,20, 128,20, 127,59,123,30, 116,81, 116,39, 115,80, 114,79, 81,77, 76,26, 76,15, 74,20, 73,79, 73,64, 73,49, 72,84, 72,16, 71,34, 70,29, 69,89, 68,83, 67,96, 63,71, 63,14, 58,22, 56,62, 56,19, 54,47, 54,42, 54,37, 41,90, 36,87 und 20,93; und
(e) die MK05-Fraktion hat den Anschein eine mit Zuckern konjugierte aromatische Verbindung zu sein.

3. Pharmazeutische Zusammensetzung nach Anspruch 1, wobei die bioaktive JB01A-Fraktion die folgenden physikalisch chemischen Eigenschaften aufweist
(a) weiß gefärbter Feststoff, der sowohl DMSO als auch in Wasser löslich ist;
(b) die TLC zeigt in dem Lösungsmittelsystem n-Butanol-Essigsäure-Wasser (9:5:7) einen einzigen Fleck mit einem Rf von 0,07 auf;
(c) die HPLC zeigt mit dem Lösungsmittelsystem Wasser-Methanol (1:9), einer Flussrate von 0,5 ml/min, bei einem Nachweis bei 217 nm und einer analytischen Säule Intersil ODS-3 einen einzigen Ausschlag mit einer Verzögerungszeit von 8,4 min (JB01A, Ausschlag-1) auf;
(d) ¹³CNMR, ppm (125 MH₂, D₂O): 109,71, 108,417, 107,94, 104,17, 100,27, 84,38, 81,79, 81,53, 80,74, 77,03, 75,58, 73,85, 73,42, 71,71, 71,27, 70,74, 70,38, 69,12, 61,71, 61,00 und 60,54 ; und
(e) die JB01A-Fraktion ist ein Oligosaccharid;
die bioaktive Fraktion JB01B weist die folgenden physikalisch chemischen Eigenschaften auf:
(a) weiß gefärbter Feststoff, der das sowohl in DMSO als auch in Wasser löslich ist;
(b) die TLC zeigt in dem Lösungsmittelsystem n-Butonol-Essigsäure-Wasser (9:5:7) einen einzigen Ausschlag mit einem Rf von 0,27 auf;
(c) die HPLC zeigt unter den genannten Säulenbedingungen einen einzigen Ausschlag mit einer Verzögerungszeit von 8,8 min auf;
(d) ¹³CNMR, ppm (125 MH₂, D₂O): 169,48, 104,30, 98,86, 98,55, 92,60, 81,82, 76,98, 76,07, 74,57, 73,52, 73,23, 71,78, 71,46, 70,02, 69,84, 69,74, 69,30, 68,92, 68,77, 67,01, 66,43, 62,95, 62,02, 61,61, 48,67, 44,62, 38,88; und
(e) die J01B-Fraktion ist ein Oligosaccharid-Derivat; und
die bioaktive Fraktion JB01C weist die folgenden physikalisch chemischen Eigenschaften auf:
(a) weißer Feststoff, der sowohl in DMSO als auch in Wasser löslich ist;
(b) die TLC zeigt in dem Lösungsmittelsystem n-Butanol-Essigsäure-Wasser (9:5:7) einen einzigen Fleck mit einem Rf von 0,34 auf;
(c) die HPLC zeigt unter den oben genannten Bedingungen einen einzigen Ausschlag mit einer Verzögerungszeit von 0,8 min auf (JB01C, Peak-3);
(d) NMR (300 MH₂, D₂O): 2,34, 3,27, 3,28, 3,44- 3,47, 3,51, 3,60 - 3,63, 3,68, 3,92-3,99, 4,08, 4,92 und 5,36; und
(e) die JB01C Fraktion ist ein Oligosaccharid.

4. Zusammensetzung nach Anspruch 1, wobei der pharmazeutisch verträgliche Inhaltsstoff unter Nährstoffen wie Proteinen, Kohlenhydraten, Zucker, Talg, Magnesiumstearat, Zellulose, Calciumcarbonat, Stärke-Gelatinepaste und/oder Trägermaterialien, Arzneistoffträgem, Verdünnungsmitteln oder Lösungsmitteln ausgewählt ist.

5. Zusammensetzung nach Anspruch 1, wobei die besagte Zusammensetzung durch Inhalation, oral, intravenös intramuskulär, subkutan oder auf andere Art und Weise verabreicht wird.

6. Zusammensetzung nach Anspruch 1, wobei die orale Verabreichung in Form von Kapseln, Sirup, Konzentrat, Puder oder Körnchen erfolgt.

7. Zusammensetzung nach Anspruch 1, wobei die intravenös verabreichte Menge geringer ist als diejenige der oralen Verabreichung.

8. Zusammensetzung nach Anspruch 1, welche mit einem Dosierungsgrad zwischen 1 bis 20 mg/kg des Körpergewichtes wenigstens einmal am Tag über eine Dauer von wenigstens vier Wochen verabreicht wird.

9. Zusammensetzung nach Anspruch 1, wobei die Zusammensetzung aus bioaktiven Fraktionen MK03, MK04 und/oder MK05 besteht.

10. Zusammensetzung nach Anspruch 1, wobei die Zusammensetzung aus bioaktiven Fraktionen JB01C, MK03, MK04 und/oder MK05 besteht.

11. Zusammensetzung nach Anspruch 1, wobei die Menge der intravenös verabreichten bioaktiven Fraktionen geringer ist als diejenige der oralen Verabreichung.

12. Zusammensetzung nach Anspruch 1, wobei der Anteil an bioaktiven Fraktionen der M.k. gleich oder größer ist als derjenige der bioaktiven Fraktion der (PB) Piper betle.

13. Zusammensetzung nach Anspruch 1, wobei die Zusammensetzung mit einem Dosierungsgrad zwischen 0,5 bis 10,0 mg/kg des Körpergewichts wenigstens einmal am Tag über eine Dauer von vier Wochen hinweg in Abhängigkeit der Atmungsbedingungen verabreicht wird.

14. Zusammensetzung nach Anspruch 1, wobei die Zusammensetzung über eine Dauer von wenigstens vier Wochen bis zu drei Monaten verabreicht wird.

15. Zusammensetzung nach Anspruch 1, wobei die Kombination der bioaktiven Fraktionen MK03, MK04 und MK05, die von den Blättern oder anderen Pflanzenteilen der Murrya koenigi zum Blockieren der 5-Lipooxygenase-Aktivität erhalten wurde, zur Inhibierung der Leukotrien-Synthese, zur Unterdrückung der Erzeugung von Interleukin-4 und zum Immunmodulator des Typs Th1 führt.

16. Zusammensetzung nach Anspruch 1, wobei die Kombination der bioaktiven Fraktionen MK03, MK04 und MK 05, die aus Blättern oder anderen Pflanzenteilen der Murryae koenigi (M.k.) erhalten wurden, und JB01C, das aus den Blättern oder anderen Pflanzenteilen des Piper betle stammt, zum Blockieren der 5-Lipooxygenase-Aktivität, zur Inhibierung der Leukotrien-Synthese, zur Unterdrückung der Erzeugung von Interleukin-4 und zur Steigerung der Abgabe von Gamma Interferon führt.

17. Zusammensetzung nach Anspruch 1, wobei diese zur Behandlung bronchialer Atemzustände verwendet wird.

18. Zusammensetzung nach Anspruch 1, wobei diese zur Behandlung von Säugetieren und Tieren verwendet wird.

19. Zusammensetzung nach Anspruch 1, wobei diese zum Verschieben der Th2-Reaktion zur Th1-Reaktion verwendet wird.

20. Zusammensetzung nach Anspruch 1, wobei diese zur Inhibierung der durch 5-Lipooxgenase vermittelten Oxidation der Arachidonsäure in mit Neutrophilen angereicherten Bestandteilen des Vollblutes verwendet wird.

21. Zusammensetzung nach Anspruch 1, wobei diese zur Steigerung der IFN-Gamma- Reaktion und zur Herabsetzung der IL-4-Reaktion im Vollblut ex-vivo verwendet wird.

22. Zusammensetzung nach Anspruch 1, wobei diese zur Steigerung IFN-Gamma-Reaktion im einkemigen menschlichen Vollblut ex-vivo verwendet wird.

23. Zusammensetzung nach Anspruch 1, wobei diese zur Herabsetzung der IL-4-Reaktion in peripheren einkernigen Zellen des menschlichen Vollblutes verwendet wird.

## Revendications

1. Composition pharmaceutique synergétique utile comme inhibiteur du leukotriène, inhibiteur synthèse d'IL4 et immunomodulateur de type Th1, ladite composition comprenant des quantités effectives d'extraits lyophilisés, aqueux ou alcooliques obtenus des feuilles de *Piper betle* (PB) et *Murrya koenigii (M.k)* ou ses fractions bioactives JB01A, JB01B, JB01C, et MK03, MK04, MK05 respectivement dans toutes les combinaisons possibles, dérivées de ces extraits avec facultativement un ou plusieurs extraits pharmaceutiquement acceptables, dans laquelle le rapport de l'extrait de *PB* ou des fractions bioactives dérivées de cette extrait à l'extrait de *M*.*k* ou aux fractions bioactives dérivées de cet extrait est dans l'intervalle de 1:1 à 1:5.

2. Composition pharmaceutique selon la revendication 1, dans laquelle la fraction bioactive dénommée MK03 présente les caractéristiques physicochimiques suivantes :
(a) solide sec, point de fusion de 98 à 100°C, soluble dans le DMSO ;
(b) la chromatographie en couche mince présente un seul point ayant Rf 0,48 dans un des systèmes de solvants-chloroforme et méthanol (19:1) ;
(c) l'analyse CLHP présente un seul pic avec un temps de rétention de 8,5 min à un débit de 0,5 ml/min au moyen d'une colonne analytique ODS-3 intersel (4,6 x 250 mm), le système de solvants de méthanol et la détection est réalisée à 210 nm. CLHP : Colonne ODS-3 (4,6x250 mm) Débit - 0,5 ml/min ; Pic à 210 nm ; Temps de rétention-3,5 min ; Système de solvants - Méthanol ;
(d) ¹³CNMR, ppm (125 MH₂, CDCl₃) : 153,34, 153,29, 148,94, 140,73, 134,78, 131,58, 128,61, 124,22, 120,36, 119,97, 118,23, 118,02, 117,39, 116,63, 108,36, 104,39, 97,16, 78,03, 40,68, 25,67, 25,60, 22,70, 17,53 et 15,97 ;
(e) la fraction « MK03 » semble être un composé azoté pur ;
la fraction bioactive dénommée MK04 présente les caractéristiques physicochimiques suivantes :
(a) matériau gommé de couleur foncée, soluble dans du diméthylsulfoxyde ;
(b) la chromatographie en couche mince présente un seul point ayant Rf 0,38 dans le système de solvants de chloroforme et de méthanol (19:1) ;
(c) l'analyse CLHP du matériau bioactif au moyen d'une colonne analytique ODS-3 intersil, un système de solvants de méthanol et un débit de 1,0 ml/min, la détection à 254 nm montre un pic avec un temps de rétention de 5,69 min ;
(d) NMR(300 MH₂, CDCl₃) 0,94, 1,30, 1,60, 2,04,-2,10, 2,27-2,34, 2,78-2,82, 3,53-3,81, 3,85-3,92, 4,00, 4,24-4,26, et 5,26-5,41 ; et
(e) la fraction MK04 est un glycolipide
et la fraction bioactive dénommée MK05 présente les caractéristiques physicochimiques suivantes :
(a) solide de couleur foncée soluble dans le DMSO et dans l'eau ;
(b) la chromatographie en couche mince présente un seul point ayant Rf 0,66 dans le système de solvants de n-butanol-acide acétique-eau (9:5:7) ;
(c) l'analyse CLHP de cette fraction présente un pic au temps de rétention de 21 min, dans le système de solvants de méthanol-eau (1:9), débit de 0,5 ml/min et la détection à 217 nm ;
(d) ¹³CNMR, ppm (125 MH₂, D₂O) : 175,82, 169,22, 159,00, 147,63, 147,02, 144,76, 135,72, 131,28, 131,10, 129,90, 129,63, 129,20, 128,20, 127,59, 123,30, 116,81, 116,39, 115,80, 114,79, 81,77, 76,26, 76,15, 74,20, 73,79, 73,64, 73,49, 72,84, 72,16, 71,34, 70,29, 69,89, 68,83, 67,96, 63,71, 63,14, 58,22, 56,62, 56,19, 54,47, 54,42, 54,37, 41,90, 36,87 et 20,93 ; et
(e) la fraction MK05 semble être un composé aromatique conjugué avec des sucres.

3. Composition pharmaceutique selon la revendication 1, dans laquelle la fraction bioactive JB01A présente les propriétés physicochimiques suivantes :
(a) matériau solide de couleur blanche et soluble à la fois dans le DMSO et dans l'eau ;
(b) la chromatographie en couche mince présente un seul point ayant Rf 0,07 dans le système de solvants n-Butanol-acide acétique-eau (9:5:7) ;
(c) l'analyse CLHP présente un pic au temps de rétention de 8,4 min (JB01A, pic-1) avec le système de solvants eau-méthanol (1:9), débit 0,5 ml/min, détection à 217 nm et colonne analytique ODS-3 intersil ;
(d) ¹³CNMR, ppm (125 MH₂, D₂O) : 109,71, 108,417, 107,94, 104,17, 100,27, 84,38, 81,79, 81,53, 80,74, 77,03, 75,58, 73,85, 73,42, 71,71, 71,27, 70,74, 70,38, 69,12, 61,71, 61,00 et 60,54 ; et
(e) la fraction JB01A est un oligosaccharide ;
la fraction bioactive JB01B présente les caractéristiques physicochimiques suivantes :
(a) solide de couleur blanche soluble à la fois dans DMSO et dans l'eau ;
(b) la chromatographie en couche mince présente un seul point ayant Rf 0,27 dans le système de solvants n-butanol-acide acétique-eau (9:5:7) ;
(c) l'analyse CLHP présente un pic, au temps de rétention de 8,8 min (JB01B, pic-2) avec les mêmes conditions de colonne qu'indiquées ci-dessus ;
(d) ¹³CNMR, ppm (125 MH₂, D₂O) : 169,48, 104,30, 98,86, 98,55, 92,60, 81,82, 76,98, 76,07, 74,57, 73,52, 73,23, 71,78, 71,46, 70,02, 69,84, 69,74, 69,30, 68,92, 68,77, 67,01, 66,43, 62,95, 62,02, 61,61, 48,67, 44,62, 38,88; et
(e) la fraction JB01B est un dérivé d'un oligosaccharide ;
et la fraction bioactive JB01C présente les propriétés physicochimiques suivantes :
(a) solide blanc soluble à la fois dans le DMSO et dans l'eau ;
(b) la chromatographie en couche mince présente un seul point ayant Rf 0,34 dans le système de solvants n-butanol-acide acétique-eau (9:5:7) ;
(c) l'analyse CLHP présente un seul pic au temps de rétention de 9,8 min (JB01C, pic-3) avec les mêmes conditions qu'indiquées ci-dessus ;
(d) NMR (300 MH₂, D₂O) 2,34, 3,27, 3,28, 3,44-3,47, 3,51, 3,60-3,63, 3,68, 3,92-3,99, 4,08, 4,92 et 5,32 ; et
(e) la fraction JB01C est un oligosaccharide.

4. Composition selon la revendication 1, dans laquelle l'ingrédient pharmaceutiquement acceptable utilisé est choisi parmi des substances nutritives telles que les protéines, les hydrates de carbone, le sucre, le talc, le stéarate de magnésium, la cellulose, la carbonate de calcium, la pâte d'amidon-gélatine et/ou des véhiculeurs, des excipients, des diluants ou des solvants.

5. Composition selon la revendication 1, dans laquelle ladite composition est administrée par inhalation, par voie orale, intraveineuse, intramusculaire, sous-cutanée et toute autre voie appropriée.

6. Composition selon la revendication 1, dans laquelle la voie orale est sous la forme de capsules, sirops, concentrés, poudres ou granulés.

7. Composition selon la revendication 1, dans laquelle la quantité administrée par voie intraveineuse est inférieure à celle administrée par voie orale.

8. Composition selon la revendication 1, qui est administrée à une dose entre 1 à 20 mg/kg de poids corporel au moins une fois par jour pendant au moins 4 semaines.

9. Composition selon la revendication 1, dans laquelle la composition comprend les fractions bioactives MK03, MK04 et/ou MK05.

10. Composition selon la revendication 1, dans laquelle la composition comprend les fractions bioactives JB01C, MK03, MK04 et/ou MK05.

11. Composition selon la revendication 1, dans laquelle la quantité de fractions bioactives administrées par voie intraveineuse est inférieure à celle de la voie orale.

12. Composition selon la revendication 1, dans laquelle la proportion de fractions bioactives de *M.k* est égale ou supérieure à celle de la fraction bioactive de (PB) *piper betle*.

13. Composition selon la revendication 1, dans laquelle la composition est administrée à une dose entre 0,5 et 10,0 mg/kg de poids corporel au moins une fois par jour pendant au moins 4 semaines en fonction des conditions respiratoires.

14. Composition selon la revendication 1, dans laquelle la composition est administrée pendant au moins 4 semaines et au plus jusqu'à 3 mois.

15. Composition selon la revendication 1, dans laquelle la combinaison des fractions bioactives MK03, MK04 et MK05 obtenues des feuilles et d'autres parties de la plante de *Murrya Koenigi* pour bloquer l'activité de la 5-lipoxygénase entraîne l'inhibition de la synthèse du leukotriène, la suppression de la production de l'interleukine-4 et comme immunomodulateur de type Th1.

16. Composition selon la revendication 1, dans laquelle la combinaison des fractions bioactives MK03, MK04 et MK05 obtenues des feuilles et d'autres parties de la plante de *Murrya Koenigi* (*M.k*) et JB01C obtenue des feuilles d'autres parties de la plante de *piper betle* pour bloquer l'activité de la 5-lipoxygénase entraîne l'inhibition de la synthèse du leukotriène, la suppression de la production de l'interleukine-4 et pour l'amélioration de la libération de l'interféron gamma.

17. Composition selon la revendication 1, utilisée pour le traitement de conditions respiratoires bronchiques.

18. Composition selon la revendication 1, utilisée pour le traitement de mammifères et d'animaux.

19. Composition selon la revendication 1, utilisée pour décaler la réponse Th2 à la réponse Th1.

20. Composition selon la revendication 1, utilisée pour inhiber l'oxydation de l'acide arachidonique provoquée par la 5-lipoxygénase dans un composant enrichi aux neutrophiles de sang complet.

21. Composition selon la revendication 1, utilisée pour améliorer la réponse IFN-gamma et réduire la réponse IL-4 ex-vivo dans du sang humain complet.

22. Composition selon la revendication 1, utilisée pour améliorer la réponse IFN-gamma in vivo dans du sang humain complet mononucléaire (PMN).

23. Composition selon la revendication 1, utilisée pour réduire la réponse IL-4 dans des cellules mononucléaires de sang complet périphérique.
